# EUROPÄISCHE PATENTSCHRIFT

(11) **EP 2 001 416 B1**
(45) Veröffentlichungstag und Bekanntmachung des Hinweises auf die Patenterteilung: **16.05.2012**
(21) Anmeldenummer: 07723621.4
(22) Anmeldetag: 27.03.2007
(51) Int. Cl.: A61F 2/44

(54) **ZWISCHENWIRBELIMPLANTAT**
INTERVERTEBRAL IMPLANT
IMPLANT INTERVERTÉBRAL

(30) Priorität: 06.04.2006 DE 102006016985
(43) Veröffentlichungstag der Anmeldung: 17.12.2008
(73) Patentinhaber: Aesculap AG, 78532 Tuttlingen (DE)
(72) Erfinder: BEGER, Jens, 78532 Tuttlingen (DE)
(74) Vertreter: Hoeger, Stellrecht & Partner Patentanwälte
(86) Internationale Anmeldenummer: PCT/EP2007/002675
(87) Internationale Veröffentlichungsnummer: WO 2007/115676

(56) Entgegenhaltungen:
- WO-A-2004/103226
- WO-A-2005/011522
- WO-A-2005/112834
- US-A1- 2004 215 342

## Beschreibung

Die Erfindung betrifft ein Zwischenwirbelimplantat mit mindestens einer aus mehreren Teilen erzeugten Anlagefläche für einen Wirbelkörper, welche Teile relativ zueinander zwischen einer Einführposition, in der das Zwischenwirbelimplantat einen kleinen Querschnitt aufweist, und einer Arbeitsposition bewegbar sind, in der das Zwischenwirbelimplantat einen größeren Querschnitt aufweist und in der die Teile gemeinsam die Anlagefläche ausbilden, und mit einer Verstelleinrichtung zur Bewegung der Teile aus der Einführposition in die Arbeitsposition.

Bei Zwischenwirbelimplantaten, die beispielsweise als Bandscheibenersatz dienen, ist es wichtig, daß das Zwischenwirbelimplantat mit einer möglichst grossen Anlagefläche an den benachbarten Wirbelkörpern anliegt, um die auftretenden Druckkräfte möglichst gering zu halten und dadurch ein Einbrechen oder Einsintern des Zwischenwirbelimplantates in den Wirbelkörper zu vermeiden. Vorzugsweise ist die Größe der Anlagefläche so zu wählen, daß sie gleich oder nur geringfügig kleiner ist als die Stirnfläche der anliegenden Wirbelkörper.

Andererseits führt die relativ große Ausbildung der Anlagefläche zu Schwierigkeiten beim Einführen des Implantates in den Körper, insbesondere bei der Einführung über einen dorsalen oder dorsal-lateralen Zugang.

Es ist daher bekannt, die Anlagefläche bei Zwischenwirbelimplantaten aus mehreren Teilen aufzubauen, die relativ zueinander bewegbar sind und die in einer Einführposition so angeordnet sind, daß der Querschnitt des Zwischenwirbelimplantates relativ klein ist, so daß das Implantat in dieser Einführposition der Teile auch durch einen kleinen Körperzugang in den Körper eingeführt werden kann. Im Inneren des Körpers werden die Teile dann in eine Arbeitsposition bewegt, in der sie die endgültige und größere Anlagefläche aufspannen. Dabei erhält das Implantat insgesamt einen größeren Querschnitt, dies ist aber nicht störend, da dieser größere Querschnitt erst nach der Positionierung des Implantates im Inneren des Körpers eingenommen wird.

Schwierig ist allerdings die Bewegung der Teile im Körperinneren aus der Einführposition in die Arbeitsposition. Diese Bewegung muß beispielsweise durch in den Körper eingeführte Instrumente vorgenommen werden, und dies ist schwierig, und wegen der dabei aufgewendeten Kräfte können dabei auch Verletzungen auftreten.

Es ist auch bekannt, bei einem Zwischenwirbelimplantat mit bewegbaren Teilen zur Vergrößerung der Anlagefläche am Implantat eine Verstelleinrichtung vorzusehen, die im bekannten Fall mit einem Spindeltrieb arbeitet (WO 2004/103226). Allerdings wird dadurch der Aufbau des Implantates kompliziert, und dies führt auch zu vergrößerten Abmessungen.

Der nächstliegende Stand der Technik wird in WO 2005/112834 beschrieben.

Es ist Aufgabe der Erfindung, eine Möglichkeit zur Bewegung der Teile der Anlagefläche anzugeben, bei der der Aufbau des Implantates relativ einfach bleibt und bei der die Abmessungen des Implantates möglichst wenig vergrößert werden.

Diese Aufgabe wird bei einem Zwischenwirbelimplantat der eingangs beschriebenen Art erfindungsgemäß dadurch gelöst, daß die Verstelleinrichtung mindestens einen Schwellkörper aufweist, der bei Befüllung mit einem fließfähigen Medium sein Volumen vergrößert und dadurch die Teile in die Arbeitsposition bewegt.

Ein solcher Schwellkörper, der beispielsweise die Form eines Balges oder eines Ballons haben kann, läßt sich in einfacher Weise im Implantat anordnen, im ungefüllten Zustand nimmt ein solcher Schwellkörper ein minimales Volumen ein, so daß die Baugröße des Implantates praktisch unverändert bleiben kann. Durch Befüllung des Schwellkörpers mit einem fließfähigen Medium, beispielsweise einem Gas oder einer Flüssigkeit, insbesondere mit einer Kochsalzlösung, kann das Volumen des Schwellkörpers vergrößert werden, und dieser vergrößerte Schwellkörper bewegt durch seine Volumenvergrößerung die Teile der Anlagefläche aus der Einführposition in die Arbeitsposition. Dabei sind verschiedene kinematische Anordnungen möglich, beispielsweise können die Teile parallel zueinander verschiebbar sein, der Schwellkörper verschiebt dann die Teile bei der Volumenvergrößerung entsprechend. Bei einem anderen Ausführungsbeispiel können Teile durch den Schwellkörper gegeneinander verschwenkt werden.

Nach der Bewegung der Teile in die Arbeitsposition müssen die Teile in dieser Position festgelegt werden, damit sie ihre Stützfunktion im gesamten Bereich der Anlagefläche ausüben können.

Bei einer besonders bevorzugten Ausführungsform der Erfindung ist vorgesehen, daß das Zwischenwirbelimplantat neben dem Schwellkörper einen Kern aus einem quellfähigen Material aufweist, welches in einem flüssigkeitsarmen Zustand ein kleines Volumen aufweist und bei Flüssigkeitsaufnahme sein Volumen vergrößert, und daß der Kern nach Flüssigkeitsaufnahme bei vergrößertem Volumen die Teile der Anlagefläche in der Arbeitsposition derart unterstützt, daß sie an einer Rückbewegung in die Einführposition gehindert sind.

Nach dem Einführen des Zwischenwirbelimplantates in den Körper kommt das quellfähige Material mit Körperflüssigkeit in Berührung und beginnt, sein Volumen zu vergrößern. Dies kann einige Stunden dauern. In dieser Zeit werden die Teile des Implantates durch den Schwellkörper in der Arbeitsposition gehalten. Mit zunehmender Volumenvergrößerung des Kernes wird diese Stützfunktion vom Kern übernommen, der Schwellkörper kann dann entleert und / oder entfernt werden.

Das quellfähige Material des Kernes dient nicht nur der Unterstützung der Teile in der Arbeitsposition, sondern auch als Polster zwischen den Endflächen des Zwischenwirbelimplantates. Damit übernimmt das quellfähige Material eine Funktion zwischen benachbarten Wirbelkörpern, die der Funktion der entfernten Bandscheibe entspricht, die ebenfalls als Polster und Gelenk wirkt und die Wirbelkörper einerseits in geringem Umfang verschiebbar und verschwenkbar relativ zueinander lagert und andererseits als Stoßdämpfer zwischen beiden Wirbelkörpern wirkt.

Das quellfähige Material kann beispielsweise ein Hydrogel sein.

Bei einer bevorzugten Ausführungsform ist vorgesehen, daß der Schwellkörper mindestens eine Auslaßöffnung für das fließfähige Medium aufweist, die derart angeordnet ist, daß das aus ihm austretende fließfähige Medium auf das quellfähige, flüssigkeitsarme Material des Kernes auftrifft. Auf diese Weise kann durch das Austreten des fließfähigen Mediums aus der Auslaßöffnung das quellfähige Material des Kernes mit dem fließfähigen Medium beaufschlagt werden, insbesondere einer Kochsalzlösung. Dies beschleunigt die Flüssigkeitsaufnahme des quellfähigen Materials und damit seine Volumenvergrößerung.

Günstig ist es, wenn der Kern zwischen zwei Schwellkörpern angeordnet ist.

Bei einer bevorzugten Ausführungsform weist die Anlagefläche einen zentralen Anlageabschnitt und an gegenüberliegenden Seiten desselben jeweils einen schwenkbar daran gelagerten, seitlichen Anlageabschnitt auf und jedem seitlichen Anlageabschnitt ist je ein Schwellkörper zugeordnet. Man erhält damit eine sehr kompakte Konstruktion, bei der ähnlich wie bei einem Tisch mit abklappbaren Enden die Anlagefläche durch Aufklappen der seitlichen Anlageabschnitte vergrößert werden kann. Das Aufklappen erfolgt durch jeweils einen Schwellkörper, der jedem seitlichen Anlageabschnitt zugeordnet ist.

Insbesondere können die Schwellkörper und gegebenenfalls ein Kern aus quellfähigem Material an dem zentralen Anlageabschnitt festgelegt sein.

Grundsätzlich ist es möglich, daß ein Zwischenwirbelimplantat nur an einer Seite eine derartige Anlagefläche aufweist, in der Regel wird aber das Zwischenwirbelimplantat so ausgebildet sein, daß auf gegenüberliegenden Seiten des Zwischenwirbelimplantates je eine aus mehreren Teilen bestehende Anlagefläche vorgesehen ist und daß der oder die Schwellkörper und gegebenenfalls der Kern aus quellfähigem Material im Zwischenraum zwischen den beiden Anlageflächen angeordnet sind. Dies ergibt eine besonders kompakte Anordnung des Zwischenwirbelimplantates, insbesondere bei der Einführposition der Teile der Anlagefläche.

Die Schwellkörper können lösbar am Zwischenwirbelimplantat gehalten sein, so daß sie nach der Bewegung der Teile in die Arbeitsposition und nach Abstützung dieser Teile in der Arbeitsposition durch andere Mittel entfernt werden können.

Es ist auch möglich, daß die Schwellkörper aus einem resorbierbaren Material bestehen, so daß sie zunächst nach Beendigung ihrer Bewegungsarbeit und Entleerung im Körper verbleiben und dann allmählich vom Körper abgebaut werden.

Als resorbierbares Material kann beispielsweise verwendet werden Polyglykolsäure, Poly-p-Dioxanon, Copolymere aus Glykolsäure und/oder Trimethylenkarbonat und/oder Caprolakton und/oder P-Dioxanon und/oder Milchsäure. Diese Substanzen können in unterschiedlichen Gewichtsanteilen und in unterschiedlichsten Kombinationen eingesetzt werden.

Die nachfolgende Beschreibung bevorzugter Ausführungsformen der Erfindung dient im Zusammenhang mit der Zeichnung der näheren Erläuterung. Es zeigen:
- Figur 1 :: eine perspektivische Ansicht eines Wirbelkörpers und eines darauf angeordneten Zwischenwirbelimplantates mit den Teilen der Anlageflächen in der Einführposition;
- Figur 2 :: eine perspektivische Ansicht des Zwischenwirbelimplantates der Figur 1 zwischen zwei Wirbelkörpern mit den Teilen der Anlagefläche in der Arbeitsposition und mit gefüllten Schwellkörpern;
- Figur 3 :: eine Ansicht ähnlich Figur 2 mit einem Kern aus quellfähigem Material nach Flüssigkeitsaufnahme und Volumenvergrößerung;

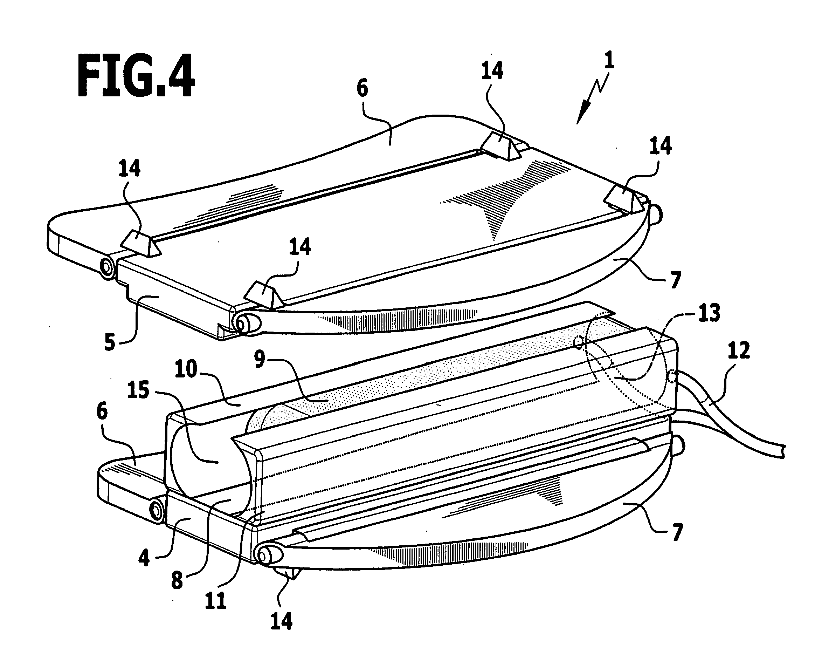

- Figur 4 :: eine perspektivische Ansicht des Zwischenwirbelimplantates der Figur 1 mit einer abgehobenen Anlagefläche, mit den Teilen der Anlagefläche in der Arbeitsposition und mit dem Kern und den Schwellkörpern vor deren Volumenvergrößerung durch Flüssigkeitsaufnahme beziehungsweise Befüllung mit einem fließfähigen Medium;
- Figur 5:: eine Teilschnittansicht längs Linie 5-5 in Figur 6 im Lagerbereich einer seitlichen Anlageplatte an einer zentralen Anlageplatte mit einer Rastvorrichtung und
- Figur 6:: eine Draufsicht auf den in Figur 5 dargestellten Schwenklagerbereich.

Das in der Zeichnung dargestellte Zwischenwirbelimplantat 1 wird als Ersatz einer entfernten Bandscheibe zwischen zwei benachbarte Wirbelkörper 2, 3 eingesetzt und stützt sich jeweils an den einander zugewandten Stirnflächen dieser beiden Wirbelkörper 2, 3 ab.

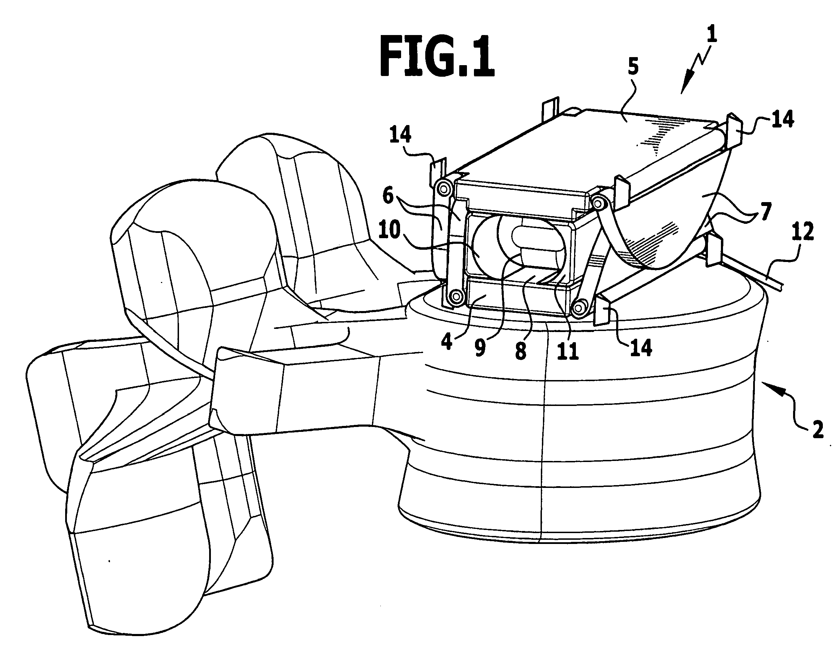

Dazu weist das Zwischenwirbelimplantat zwei parallel und im Abstand zueinander verlaufende zentrale Anlageplatten 4, 5 auf, die einen im wesentlichen rechteckigen Querschnitt aufweisen und an deren gegenüberliegenden Längsseiten um eine parallel zu den Längsseiten verlaufende Achse verschwenkbar jeweils seitliche Anlageplatten 6 und 7 gelagert sind. Jeweils eine zentrale Anlageplatte 4 oder 5 und die daran gelagerten Anlageplatten 6 und 7 bilden gemeinsam eine Endplatte des Zwischenwirbelimplantates 1 aus. Die beiden Endplatten sind dabei im wesentlichen gleich aufgebaut, sie unterscheiden sich lediglich dadurch, daß eine Endplatte geringfügig breiter ist als die andere, so daß die seitlichen Anlageplatten 6, 7 der beiden Endplatten aufeinanderliegen, wenn sie senkrecht von den zentralen Anlageplatten 4, 5 abstehen (Figur 1). Diese Anordnung, in der die seitlichen Anlageplatten 6, 7 senkrecht zu den zentralen Anlageplatten 4, 5 verlaufend aufeinander aufliegen, wird nachfolgend als Einführposition bezeichnet, in dieser Position der Anlageplatten 6, 7 ergibt sich eine besonders kleine Querschnittsfläche des Zwischenwirbelimplantates, so daß das Einführen des Implantates durch eine Körperöffnung in dieser Einführposition auch dann möglich ist, wenn kleine Einführöffnungen verwendet werden.

In dem Zwischenraum 8 zwischen den Anlageplatten 4 und 5 befindet sich in der Mitte ein sich im wesentlichen über die Länge der zentralen Anlageplatten 4, 5 erstreckender Kern 9 aus einem quellfähigen Material, der zwischen zwei sich über die gesamte Länge der zentralen Anlageplatten 4, 5 erstreckenden Schwellkörpern 10, 11 angeordnet ist, die sich zu beiden Seiten des Kernes 9 entlang der Längsseiten der zentralen Anlageplatten 4, 5 erstrecken.

Der Kern besteht aus einem quellfähigen Material, das nach Entzug von Flüssigkeit, insbesondere Wasser, ein geringes Volumen aufweist, das aber von sich aus umgebende Flüssigkeit aufnehmen kann und dabei sein Volumen vergrößert. Die Volumenvergrößerung kann anisotrop sein, das heißt bevorzugt in bestimmten Richtungen erfolgen.

Als Materialien kommen dabei prinzipiell alle nicht degradierbaren hydrophilen Polymere in Frage. Beispiele sind Polyacrylsäure und deren Derivate wie Polymethacrylsäure, Polyacrylsäureamid, Polyacrylonitril, Polyacrylsäureester, Polyhydroxyethylmethacrylate, oder auch andere Substanzen, wie beispielsweise Polyvinylpyrrolidon (PVP), Polyurethane, hochmolekularer Polyvinylalkohol.

Denkbar sind auch Polymerblends (Copolymere, welche über Bindungen miteinander verbunden sind) aus den oben genannten Polymeren oder Interpenetrating Networks (IPN) aus den oben genannten Polymeren. IPN bestehen aus mindestens zwei unterschiedlichen Polymeren, deren Polymerketten ineinander verhakt sind und über physikalische Wechselwirkungen (van der Waals-, elektrostatische, H-Brückenbindungen und / oder ionische Kräfte) miteinander verbunden sind.

Weitere Polymermischungen, welche eingesetzt werden können, sind Copolymer sowie IPN aus Polyacrylaten (Polyacrylsäure und deren Derivate wie Polymethacrylsäure, Polyacrylsäureamid, Polyacrylsäurenitril, Polyacrylsäureester) mit Polycaprolacton.

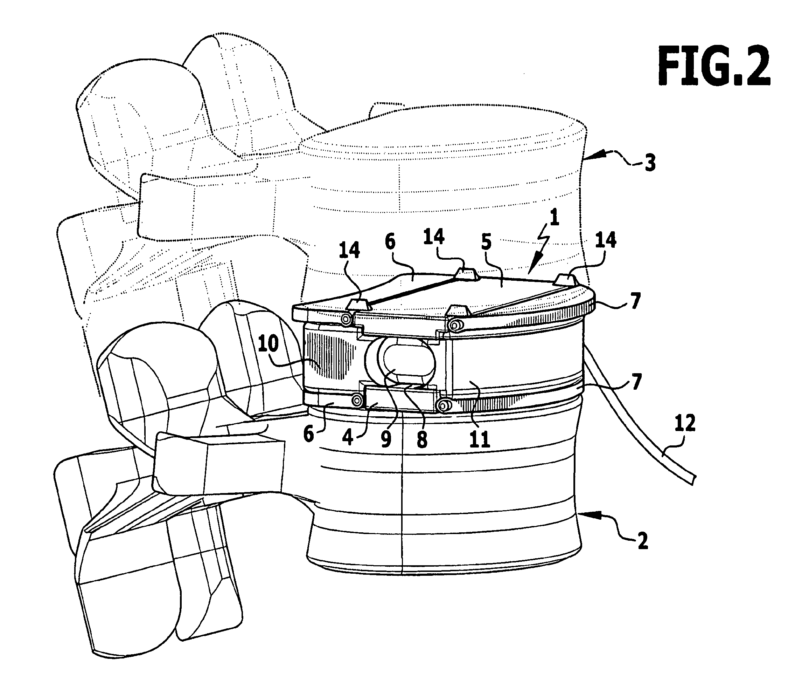

Die Schwellkörper 10, 11 sind im dargestellten Ausführungsbeispiel als ballonartige Hohlräume ausgebildet, die dicht an dem Kern 9 anliegen und die biegeschlaffe und gegebenenfalls auch dehnbare Wände aufweisen. Beide Schwellkörper 10, 11 sind mit Zufuhrleitungen 12, 13 verbunden, durch die ein fließfähiges Medium in die Schwellkörper 10, 11 eingeleitet werden kann. Es kann sich dabei um ein Gas handeln, vorzugsweise jedoch um eine Flüssigkeit, insbesondere um eine Kochsalzlösung. Durch die Füllung der Schwellkörper 10, 11 mit dem fließfähigen Medium wird das Volumen der Schwellkörper 10, 11 vergrößert, sei es durch Auffalten der Wände der Schwellkörper 10, 11, sei es durch elastische Dehnung der Wände der Schwellkörper 10, 11, sei es durch eine Kombination beider Effekte. Diese Volumenvergrößerung der Schwellkörper 10, 11 führt dazu, daß die Schwellkörper 10, 11 sich vom Kern 9 weg seitlich gegen die abgeklappten seitlichen Anlageplatten 6, 7 ausdehnen und diese dabei jeweils nach außen schwenken, bis die seitlichen Anlageplatten 6, 7 um etwa 90° nach außen geschwenkt sind und sich in einer Ebene mit den zentralen Anlageplatten 4, 5 erstrecken, an denen sie schwenkbar angelenkt sind (Figur 2). Sie bilden dann zusammen mit den zentralen Anlageplatten 4 und 5 eine Anlagefläche, die wesentlich größer ist als die Fläche der zentralen Anlageplatten 4, 5, und auch der Querschnitt des Zwischenwirbelimplantates 1 ist dann erheblich größer als bei einer Stellung der seitlichen Anlageplatten 6, 7 in der Einführposition (Figur 1).

Im gefüllten Zustand erstrecken sich die Schwellkörper 10, 11 somit über den Zwischenraum 8 zwischen den zentralen Anlageplatten 4, 5 seitlich in den Zwischenraum zwischen die aufgeklappten seitlichen Anlageplatten 6, 7 und füllen somit mit Ausnahme des Kernes 9 den gesamten Zwischenraum zwischen den Endplatten des Zwischenwirbelimplantates 1 aus.

Die Ausklappbewegung der seitlichen Anlageplatten 6, 7 wird durch Anschläge 14 begrenzt, die an den seitlichen Anlageplatten 6, 7 angeordnet sind und sich an die zentralen Anlageplatten 4, 5 anlegen, sobald die seitlichen Anlageplatten 6, 7 in einer Ebene mit den zentralen Anlageplatten 4, 5 stehen.

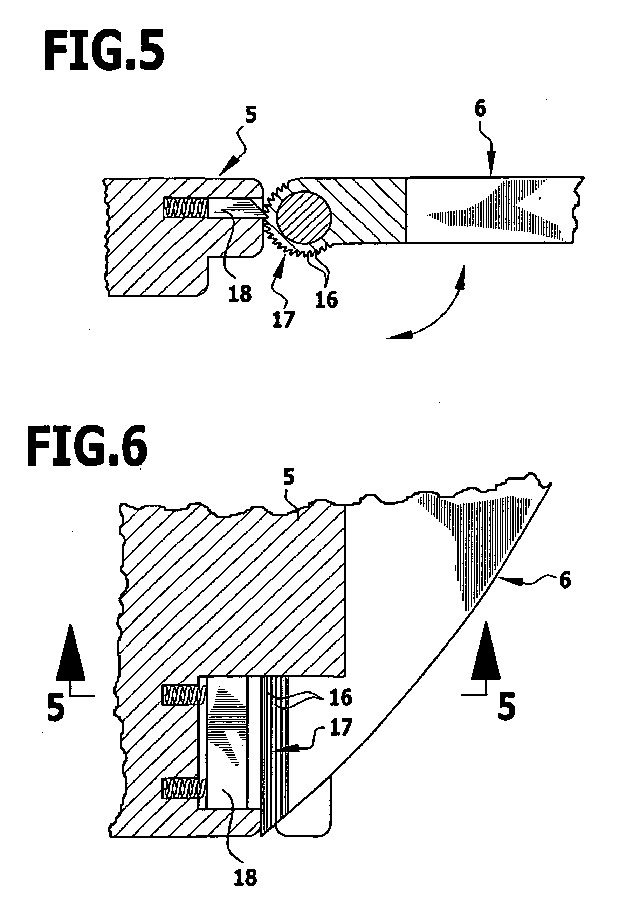

Nach dem Aufklappen der seitlichen Anlageplatten 6, 7 in die als Arbeitsposition bezeichnete Stellung, in der sie zusammen mit den zentralen Anlageplatten 4, 5 eine vergrößerte Anlagefläche bilden, müssen die seitlichen Anlageplatten 6, 7 in der aufgeklappten Stellung fixiert werden. Bei dem Ausführungsbeispiel der Figuren 5 und 6 sind dazu die seitlichen Anlageplatten 7 im Bereich der Schwenklagerung außenseitig mit einer mit Rasten 16 versehenen Rastfläche 17 ausgestattet, und in der zentralen Anlageplatte 5 sind quer zur Schwenkachse verschiebbare, federbelastete Rastelemente 18 gelagert, die an der Rastfläche 17 anliegen und beim Verschwenken der seitlichen Anlageplatte 7 an der Rastfläche 17 entlanggleiten. Dabei ist die Geometrie der Rasten 16 und der Rastelemente 18 so gewählt, daß die Teile in einer Richtung aneinander entlanggleiten können, während in der entgegengesetzten Richtung eine Verrastung durch Eingriff des Rastelementes 18 in die Rasten 16 eintritt und damit eine Verriegelung der seitlichen Anlageplatte 7. Mit anderen Worten kann jede seitliche Anlageplatte 7 nur aus der Einführposition in die Implantierposition verschwenkt werden, nicht aber in umgekehrter Richtung.

Selbstverständlich kann dieselbe Anordnung auch bei der anderen seitlichen Anlageplatte 6 verwendet werden.

Statt der Fixierung der seitlichen Anlageplatten 6, 7 durch mechanische Verriegelungsmittel oder zusätzlich zu dieser Fixierung durch die mechanischen Verriegelungsmittel kann außerdem vorgesehen sein, daß eine Fixierung der seitlichen Anlageplatten 6, 7 durch den Kern 9 erfolgt.

Dieser Kern 9 wird beim Einführen des Implantates in einem flüssigkeitsarmen Zustand eingeführt, also mit einem geringen Volumen.

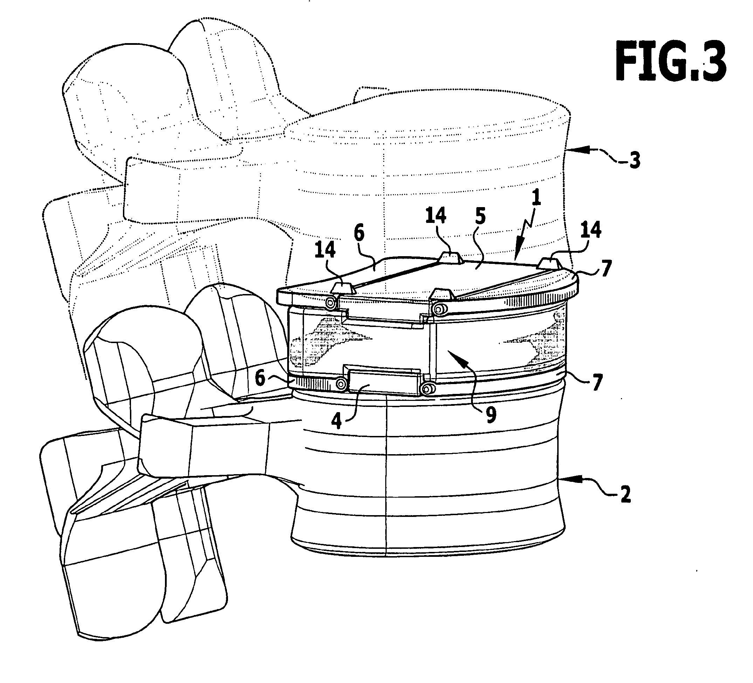

Nach dem Einführen in den Körper kommt das Material des Kernes 9 mit Umgebungsflüssigkeit in Kontakt, gegebenenfalls auch mit Flüssigkeit, die durch Austrittsöffnungen 15 aus den Schwellkörpern 10, 11 austritt. Der Kontakt mit der Flüssigkeit führt zu einer Flüssigkeitsaufnahme durch den Kern 9 und damit zu einer deutlichen Volumenvergrößerung des Kernes 9, der sich durch diese Volumenvergrößerung in den gesamten Zwischenraum zwischen den zentralen Anlageplatten 4, 5 und zwischen den aufgeklappten seitlichen Anlageplatten 6, 7 ausdehnt (Figur 3). Die Schwellkörper 10 können in diesem Zustand entleert werden, so daß sie die Ausdehnung des Kernes 9 nicht behindern, oder aber sie werden vom Operateur vollständig entfernt, da sie ihre Aufgabe als Ausschwenkeinrichtung für die seitlichen Anlageplatten 6, 7 erfüllt haben.

Nach dem Aufquellen des Kernes 9 bildet dieser zwischen den beiden ausgeklappten Endplatten ein Polster aus, welches eine gewisse Beweglichkeit der Endplatten relativ zueinander ermöglicht und gleichzeitig als Stoßdämpfer zwischen den benachbarten Wirbelkörpern 2, 3 wirkt.

Die Kombination des quellfähigen Kernes 9 und der füllbaren Schwellkörper 10, 11 ermöglicht es, trotz des zeitaufwendigen Quellvorganges des Kernes 9, der sich über Stunden erstrecken kann, die seitlichen Anlageplatten 6, 7 des Zwischenwirbelimplantates 1 während einer Operation sehr schnell in die Arbeitsposition zu überführen und dann nach Abschluß des Quellvorganges des Kernes 9 diesem die Stützfunktion der Endplatten vollständig zu überlassen, ohne daß die Schwellkörper 10, 11 noch an dieser Stützfunktion teilnehmen.

## Patentansprüche

1. Zwischenwirbelimplantat (1) mit mindestens einer aus mehreren Teilen erzeugten Anlagefläche für einen Wirbelkörper (2, 3), welche Teile relativ zueinander zwischen einer Einführposition, in der das Zwischenwirbelimplantat (1) einen kleinen Querschnitt aufweist, und einer Arbeitsposition bewegbar sind, in der das Zwischenwirbelimplantat (1) einen größeren Querschnitt aufweist und in der die Teile gemeinsam die Anlagefläche ausbilden, und mit einer Verstelleinrichtung zur Bewegung der Teile aus der Einführposition in die Arbeitsposition, **dadurch gekennzeichnet, daß** die Verstelleinrichtung mindestens einen Schwellkörper (10, 11) aufweist, der bei Befüllung mit einem fließfähigen Medium sein Volumen vergrößert und dadurch die Teile (6, 7) in die Arbeitsposition bewegt.

2. Implantat nach Anspruch 1, **dadurch gekennzeichnet, daß** der Schwellkörper (10, 11) ein Balg ist.

3. Implantat nach Anspruch 1, **dadurch gekennzeichnet, daß** der Schwellkörper (10, 11) ein Ballon ist.

4. Implantat nach einem der voranstehenden Ansprüche, **dadurch gekennzeichnet, daß** das fließfähige Medium ein Gas ist.

5. Implantat nach einem der Ansprüche 1 bis 3, **dadurch gekennzeichnet, daß** das fließfähige Medium eine Flüssigkeit ist.

6. Implantat nach Anspruch 5, **dadurch gekennzeichnet, daß** das fließfähige Medium eine Kochsalzlösung ist.

7. Implantat nach einem der voranstehenden Ansprüche, **dadurch gekennzeichnet, daß** es neben dem Schwellkörper (10, 11) einen Kern (9) aus einem quellfähigen Material aufweist, welches in einem flüssigkeitsarmen Zustand ein kleines Volumen aufweist und bei Flüssigkeitsaufnahme sein Volumen vergrößert, und daß der Kern (9) nach Flüssigkeitsaufnahme bei vergrößertem Volumen die Teile (6, 7) der Anlagefläche in der Arbeitsposition derart unterstützt, daß sie an einer Rückbewegung in die Einführposition gehindert sind.

8. Implantat nach Anspruch 7, **dadurch gekennzeichnet, daß** das quellfähige Material ein Hydrogel ist.

9. Implantat nach einem der Ansprüche 7 oder 8, **dadurch gekennzeichnet, daß** der Schwellkörper (10, 11) mindestens eine Auslaßöffnung (15) für das fließfähige Medium aufweist, die derart angeordnet ist, daß das aus ihr austretende fließfähige Medium auf das quellfähige, flüssigkeitsarme Material des Kernes (9) auftrifft.

10. Implantat nach einem der Ansprüche 7 bis 9, **dadurch gekennzeichnet, daß** der Kern (9) zwischen zwei Schwellkörpern (10, 11) angeordnet ist.

11. Implantat nach einem der voranstehenden Ansprüche, **dadurch gekennzeichnet, daß** die Anlagefläche einen zentralen Anlageabschnitt (4, 5) und an gegenüberliegenden Seiten jeweils einen schwenkbar daran gelagerten, seitlichen Anlageabschnitt (6, 7) aufweist und daß jedem seitlichen Anlageabschnitt (6, 7) je ein Schwellkörper (10, 11) zugeordnet ist.

12. Implantat nach Anspruch 11, **dadurch gekennzeichnet, daß** die Schwellkörper (10, 11) und gegebenenfalls ein Kern (9) aus quellfähigem Material an dem zentralen Anlageabschnitt (4, 5) festgelegt sind.

13. Implantat nach einem der voranstehenden Ansprüche, **dadurch gekennzeichnet, daß** auf gegenüberliegenden Seiten des Zwischenwirbelimplantates (1) je eine aus mehreren Teilen (4, 6, 7; 5, 6, 7) bestehende Anlagefläche vorgesehen ist und daß der oder die Schwellkörper (10, 11) und gegebenenfalls der Kern (9) aus quellfähigem Material im Zwischenraum zwischen den beiden Anlageflächen angeordnet sind.

14. Implantat nach einem der voranstehenden Ansprüche, **dadurch gekennzeichnet, daß** die Schwellkörper (10, 11) lösbar am Zwischenwirbelimplantat (1) gehalten sind.

15. Implantat nach einem der Ansprüche 1 bis 13, **dadurch gekennzeichnet, daß** die Schwellkörper (10, 11) aus einem resorbierbaren Material bestehen.

## Claims

1. Intervertebral implant (1) with at least one abutment surface formed from a plurality of parts for a vertebral body (2, 3), said parts being movable relative to one another between an insertion position, in which the intervertebral implant (1) has a small cross-section, and a working position, in which the intervertebral implant (1) has a larger cross-section and in which the parts jointly form the abutment surface, and with an adjusting means for moving the parts out of the insertion position into the working position, **characterised in that** the adjusting means has at least one expansion body (10, 11), which increases in volume when filled with a flowable medium and thus moves the parts (6, 7) into the working position.

2. Implant according to claim 1, **characterised in that** the expansion body (10, 11) is a bellows.

3. Implant according to claim 1, **characterised in that** the expansion body (10, 11) is a balloon.

4. Implant according to one of the preceding claims, **characterised in that** the flowable medium is a gas.

5. Implant according to one of claims 1 to 3, **characterised in that** the flowable medium is a liquid.

6. Implant according to claim 5, **characterised in that** the flowable medium is a sodium chloride solution.

7. Implant according to one of the preceding claims, **characterised in that** besides the expansion body (10, 11) it has a core (9) composed of a swellable material, which has a small volume in a state low in liquid and upon liquid absorption increases its volume, and that after liquid absorption with increased volume the core (9) supports the parts (6, 7) of the abutment surface in the working position in such a manner that they are prevented from moving back into the insertion position.

8. Implant according to claim 7, **characterised in that** the swellable material is a hydrogel.

9. Implant according to one of claims 7 or 8, **characterised in that** the expansion body (10, 11) has at least one outlet (15) for the flowable material, which is arranged in such a manner that the flowable medium discharging from it impinges against the swellable low-liquid material of the core (9).

10. Implant according to one of claims 7 to 9, **characterised in that** the core (9) is arranged between two expansion bodies (10, 11).

11. Implant according to one of the preceding claims, **characterised in that** the abutment surface has a central abutment section (4, 5) and a respective lateral abutment section (6, 7) mounted to pivot thereon on opposite sides, and that each lateral section (6, 7) has a respective associated expansion body (10, 11).

12. Implant according to claim 11, **characterised in that** the expansion bodies (10, 11) and when applicable a core (9) of swellable material are fixed to the central abutment section (4, 5).

13. Implant according to one of the preceding claims, **characterised in that** an abutment surface comprising a plurality of parts (4, 6, 7; 5, 6, 7) is respectively provided on opposite sides of the intervertebral implant (1), and that the expansion body or bodies (10, 11) and when applicable the core (9) of swellable material are arranged in the space between the two abutment surfaces.

14. Implant according to one of the preceding claims, **characterised in that** the expansion bodies (10, 11) are detachably held on the intervertebral implant (1).

15. Implant according to one of claims 1 to 13, **characterised in that** the expansion bodies (10, 11) are made of a resorbable material.

## Revendications

1. Implant intervertébral (1) comprenant au moins une surface d'appui formée de plusieurs parties et destinée à une vertèbre (2, 3), lesdites parties pouvant être déplacées relativement les unes par rapport aux autres entre une position d'insertion, dans laquelle l'implant intervertébral (1) présente une petite section, et une position de travail dans laquelle l'implant intervertébral (1) présente une section plus grande et dans laquelle les parties forment en commun la dite surface d'appui, l'ensemble comprenant également un dispositif de réglage pour déplacer les parties de la position d'insertion à la position de travail, **caractérisé en ce que** le dispositif de réglage présente au moins un corps expansible (10, 11), qui, lors d'un remplissage à l'aide d'un agent fluide, augmente de volume en déplaçant ainsi les parties (6, 7) dans la position de travail.

2. Implant selon la revendication 1, **caractérisé en ce que** le corps expansible (10, 11) est un soufflet.

3. Implant selon la revendication 1, **caractérisé en ce que** le corps expansible (10, 11) est un ballonnet.

4. Implant selon l'une des revendications précédentes, **caractérisé en ce que** l'agent fluide est un gaz.

5. Implant selon l'une des revendications 1 à 3, **caractérisé en ce que** l'agent fluide est un liquide.

6. Implant selon la revendication 5, **caractérisé en ce que** l'agent fluide est une solution physiologique (solution de chlorure de sodium).

7. Implant selon l'une des revendications précédentes, **caractérisé en ce qu'**il comprend, en-dehors du corps expansible (10, 11), un noyau (9) en un matériau susceptible de gonfler, qui présente un petit volume dans un état pauvre en liquide, et, en cas d'absorption de liquide, augmente de volume, et **en ce que** le noyau (9), après absorption de liquide, contribue, en ayant son volume augmenté, à soutenir les parties (6, 7) de la surface d' appui dans la position de travail, de manière à empêcher leur mouvement de retour à la position d'insertion.

8. Implant selon la revendication 7, **caractérisé en ce que** le matériau susceptible de gonfler est un hydrogel.

9. Implant selon l'une des revendications 7 ou 8, **caractérisé en ce que** le corps expansible (10, 11) présente au moins une ouverture de sortie (15) pour l'agent fluide, qui est agencée de manière à ce que l'agent fluide qui s'en échappe tombe sur le matériau susceptible de gonfler, pauvre en liquide, du noyau (9).

10. Implant selon l'une des revendications 7 à 9, **caractérisé en ce que** le noyau (9) est agencé entre deux corps expansibles (10, 11).

11. Implant selon l'une des revendications précédentes, **caractérisé en ce que** la surface d'appui présente un secteur d'appui central (4, 5) et, sur deux côtés opposés, respectivement un secteur d'appui latéral (6, 7), qui y est monté de manière pivotante, et **en ce qu'**à chaque secteur d'appui latéral (6, 7) est associé respectivement un corps expansible (10, 11).

12. Implant selon la revendication 11, **caractérisé en ce que** les corps expansibles (10, 11), et le cas échéant, un noyau (9) en un matériau susceptible de gonfler, sont fixés au secteur d'appui central (4, 5).

13. Implant selon l'une des revendications précédentes, **caractérisé en ce que** sur des côtés opposés de l'implant intervertébral (1) est prévu respectivement une surface d'appui constituée de plusieurs parties (4, 6, 7 ; 5, 6, 7), et **en ce que** le ou les corps expansibles (10, 11) et, le cas échéant, le noyau (9) en matériau susceptible de gonfler, sont agencés dans l'espace intermédiaire entre les deux surfaces d'appui.

14. Implant selon l'une des revendications précédentes, **caractérisé en ce que** les corps expansibles (10, 11) sont maintenus de manière démontable sur l'implant intervertébral (1).

15. Implant selon l'une des revendications 1 à 13, **caractérisé en ce que** les corps expansibles (10, 11) sont réalisés en un matériau résorbable.
